# EUROPÄISCHE PATENTSCHRIFT

(11) **EP 2 030 521 B1**
(45) Veröffentlichungstag und Bekanntmachung des Hinweises auf die Patenterteilung: **24.11.2010**
(21) Anmeldenummer: 07016807.5
(22) Anmeldetag: 28.08.2007
(51) Int. Cl.: A45C 11/00, A45C 13/02, A61M 5/00

(54) **Tasche für ein Blutzuckermessgerät**
Bag for a blood sugar measuring device
Poche pour un lecteur de glycémie

(43) Veröffentlichungstag der Anmeldung: 04.03.2009
(73) Patentinhaber: Roche Diagnostics GmbH, 68305 Mannheim (DE); F.Hoffmann-La Roche AG, 4070 Basel (CH)
(72) Erfinder: Kroll, Steffen, 06108 Halle (DE); Hoppert, Kirsten, 06108 Halle (DE)
(74) Vertreter: Mommer, Niels

(56) Entgegenhaltungen:
- GB-A- 145 977
- US-A1- 2003 038 047
- US-A1- 2006 040 333
- US-B1- 6 602 469

## Beschreibung

Die Erfindung betrifft eine Tasche für ein Blutzuckermessgerät, welche die im Oberbegriff des Anspruchs 1 angegebenen Merkmale aufweist. Eine derartige Tasche ist beispielsweise aus der WO2006/023241 A1 bekannt.

Viele Diabetiker sind gezwungen, mehrmals täglich ihren Blutzuckerspiegel zu messen und müssen deshalb ständig ein Blutzuckermessgerät sowie dafür benötigtes Verbrauchsmaterial, beispielsweise Testelemente, Batterien und/oder Lanzetten, mit sich führen. Deshalb werden Taschen für Blutzuckermessgeräte benötigt, die möglichst klein und kompakt sein sollten, um Diabetikern das Mitführen eines Messgeräts zu erleichtern.

Aufgabe der Erfindung ist es deshalb, einen Weg aufzuzeigen, wie Diabetikern das Mitführen von Blutzuckermessgeräten und evtl, benötigtes Verbrauchsmaterial möglichst weitgehend erleichtert werden kann.

Diese Aufgabe wird durch eine Tasche für ein Blutzuckermessgerät mit den im Anspruch 1 angegebenen Merkmalen gelöst. Die Aufgabe wird auch gelöst durch ein Blutzuckermesssystem mit einem Blutzuckermessgerät, Verbrauchsmaterialien und einer derartigen Tasche. Vorteilhafte Weiterbildungen der Erfindung sind Gegenstand der abhängigen Unteransprüche.

Eine erfindungsgemäße Tasche hat eine Klappe, mit welcher der Innenraum der Tasche bei Bedarf unterteilt werden kann, um Platz für Verbrauchsmaterial zu schaffen. Indem die Klappe einer erfindungsgemäßen Tasche aus einer ersten Stellung in eine zweite Stellung geschwenkt wird, kann zwischen dem verschlossenen Ende des Innenraums der Tasche und der Klappe Platz für Verbrauchsmaterial geschaffen werden. Auf der der Taschenöffnung zugewandten Seite der Klappe kann dann das Blutzuckermessgerät platziert werden.

Im Rahmen der Erfindung wurde erkannt, dass Diabetiker nur bei einer längeren Abwesenheit von Zuhause einen Vorrat von Verbrauchsmaterial für ihr Blutzuckermessgerät mitnehmen müssen. Bei kurzzeitiger Abwesenheit genügt in der Regel der in ein Blutzuckermessgerät eingelegte Verbrauchsmaterialvorrat. Herkömmliche Taschen für Blutzuckermessgeräte sind deshalb für kurzzeitige Abwesenheit eines Diabetikers von zuhause unnötig groß und sperrig, da stets ein Fach für Verbrauchsmaterial vorgesehen ist, obwohl dieses Fach nur bei längeren Reisen benötigt wird.

Eine erfindungsgemäße Tasche schafft hier Abhilfe. Wenn in der Tasche kein Verbrauchsmaterial mitgeführt werden sollen, befindet sich die Klappe in ihrer ersten Stellung, so dass das Blutzuckermessgerät entsprechend tiefer in die Tasche hineingeschoben werden kann und die Tasche folglich weniger Platz wegnimmt. Wenn Verbrauchsmaterial mitgeführt werden soll, wird dieses in die Tasche eingelegt und kann mittels der in die zweite Stellung gebrachten Klappe ordentlich zwischen dem verschlossenen Ende der Tasche und der Klappe verstaut werden. Zwischen der Klappe und der Öffnung des Innenraums der Tasche kann dann das Blutzuckermessgerät eingelegt und ebenfalls sicher verstaut werden.

Im Rahmen der Erfindung wurde erkannt, dass es keinen Nachteil bedeutet, wenn auf in der Tasche enthaltenes Verbrauchsmaterial erst zugegriffen werden kann, nachdem ein auf der anderen Seite der Klappe platziertes Messgerät entnommen wurde. Verbrauchsmaterial wird nämlich in der Regel nur zusammen mit dem Messgerät benötigt, beispielsweise um darin eingelegt zu werden.

Bevorzugt ist der Abstand, in dem die Klappe von dem geschlossenen Ende an einer der gegenüberliegenden Seiten befestigt ist, größer als der Abstand zwischen einem Klappenrand, mit dem die Klappe befestigt ist und einem gegenüberliegenden Klappenrand. Auf diese Weise kann die Klappe in der ersten Stellung zu dem geschlossenen Ende des Innenraums hin geschwenkt sein. Wenn ein Blutzuckermessgerät in eine ansonsten leere Tasche hineingeschoben wird, gelangt die Klappe dann von selbst in diese Stellung gelangt, in der sie nur einen minimalen Platzbedarf verursacht.

Bevorzugt liegt die Klappe in der zweiten Stellung mit ihrem Rand der Taschenseite an, die der die Klappe tragenden Seite gegenüberliegt.

Bei einer erfindungsgemäßen Tasche können zwischen den einander gegenüberliegenden Seiten, welche den Innenraum begrenzen, weitere Seiten vorgesehen sein, so dass sich beispielsweise ein viereckiger Querschnitt des Innenraums ergeben kann. Bevorzugt ist aber, dass sich die einander gegenüberliegenden Seiten an ihren Rändern berühren. Beispielsweise können die Ränder miteinander verschweißt, verklebt oder insbesondere auch vernäht sein.

Vorzugsweise grenzen die einander gegenüberliegenden Seiten, die den Innenraum der Tasche begrenzen, an dem verschlossenen Ende des Innenraums fugenlos aneinander an. Dies kann am einfachsten dadurch erreicht werden, dass die Tasche aus einem Streifen hergestellt wird, der gefaltet wird, so dass sich seine Längsränder auf jeweils einer Seite aufeinander legen und dort miteinander verbunden werden. Das verschlossene Ende des Innenraums kann so als Faltkante des Streifens ausgebildet werden. Zwar ist es auch möglich, zur Herstellung der Tasche zwei beispielsweise rechteckige Stoffstreifen aufeinander zu legen und umlaufend zu verbinden, so dass lediglich eine Öffnung bleibt, jedoch ist dies aufwendiger, da auf diese Weise eine größere Verbindungslänge entsteht.

Besonders bevorzugt ist, dass die einander gegenüberliegenden Seiten zwischen ihren Rändern eine unterschiedliche Breite haben. Vorzugsweise hat jene Seite, an welcher die Klappe befestigt ist, eine geringere Breite, beispielsweise nur 97 % oder weniger der Breite der gegenüberliegenden Seite. Dies bewirkt, dass die Seite, an welcher die Klappe befestigt ist, näherungsweise eben bleibt, während sich die gegenüberliegende Seite wegen ihrer größeren Breite von selbst nach außen wölbt, so dass der Innenraum der Tasche einen näherungsweise D-förmigen oder trapezförmigen Querschnitt hat.

Der Innenraum der Tasche und seine Öffnung haben vorzugsweise eine sich entlang der gegenüberliegenden Seiten erstreckenden Breite, welche die sich in dieser Richtung erstreckende Breite der Klappe übersteigt. Diese Maßnahme hat den Vorteil, dass sich die Klappe leichter verschwenken lässt. Besonders günstig ist es, wenn sich der Innenraum an beiden Enden der Klappe über diese hinaus erstreckt, beispielsweise um 2 mm oder mehr, insbesondere 3 mm oder mehr.

Eine erfindungsgemäße Tasche hat vorzugsweise eine Klapplasche, die zum Verschließen des Innenraums über dessen Öffnung geklappt werden kann. Mit einer solchen Klapplasche lässt sich die effektive Größe einer erfindungsgemäßen Tasche besonders einfach daran anpassen, ob in ihr nur ein Blutzuckermessgerät oder zusätzlich auch Verbrauchsmaterial enthalten ist. Enthält die Tasche nämlich nur ein Blutzuckermessgerät, so lässt sich dieses tiefer, vorzugsweise bis zum verschlossenen Ende des Innenraums, in die Tasche hinein schieben. Enthält die Tasche auch Verbrauchsmaterial, kann das Blutzuckermessgerät weniger weit in die Tasche hinein geschoben werden und ragt unter Umständen sogar etwas aus der Öffnung des Innenraums hinaus. Eine biegsame Klapplasche passt sich von selbst an derart unterschiedliche Verhältnisse an, indem sie sich bevorzugt um eine Biegekante biegt, deren Abstand von der Öffnung variabel, je nach aktuellen Anforderungen gewählt werden kann. Die Klapplasche kann auf diese Weise je nach Füllzustand der Tasche mit einem mehr oder weniger langen Abschnitt an einer der einander gegenüberliegenden Taschenseiten, die den Innenraum begrenzen anliegen.

Bevorzugt ist die Klapplasche in verschiedenen Schließstellungen über die Öffnung klappbar, wobei sich die Schließstellungen dadurch unterscheiden, wie weit sich die Klapplasche entlang der einander gegenüberliegenden Seiten erstreckt. Vorzugsweise erstreckt sich die Klapplasche in einer ersten Schließstellung mindestens entlang von zwei Dritteln der Länge der gegenüberliegen Seiten, gemessen von der Öffnung bis zu dem verschlossenen Ende des Innenraums, und in einer zweiten Schließstellung höchstens entlang von einer Hälfte der Länge der gegenüberliegen Seiten. Besonders bevorzugt erstreckt sich die Klapplasche in einer ersten Schließstellung mindestens entlang von drei Vierteln der Länge der gegenüberliegen Seiten und in einer zweiten Schließstellung höchstens entlang von einem Drittel der Länge der gegenüberliegen Seiten.

Bevorzugt ist die Klapplasche ebenso breit, wie die den Innenraum begrenzende Taschenseite, von der die Klapplasche ausgeht. Bevorzugt hat die Klapplasche gemessen von der Öffnung bis zu ihrem freien Ende eine Länge eine größere. Länge als die einander gegenüberliegenden Taschenseiten, die den Innenraum begrenzen, wobei die Länge der Taschenseiten von der Öffnung der Tasche bis zu dem geschlossenen Ende des Innenraums zu messen ist. Bevorzugt beträgt die Länge der Klapplasche 8 cm bis 15 cm, besonders bevorzugt 10 cm bis 13 cm, insbesondere 11 cm bis 12,5 cm.

Die Klapplasche kann bei geschlossener Tasche beispielsweise mit einer Schlaufe fixiert werden. Bevorzugt ist die Schlaufe an der Klapplasche angebracht. Zum Fixieren der Klapplasche kann die Schlaufe über die einander gegenüberliegenden Seitenwände, die den Innenraum begrenzen, gestülpt werden. Die Schlaufe kann beispielsweise ein gummielastisches Band, insbesondere ein Textilband, sein.

Bevorzugt beträgt die Länge des Innenraums der Tasche, gemessen von der Öffnung bis zu seinem verschlossenen Ende, nicht mehr als 12 cm, bevorzugt nicht mehr als 11 cm, insbesondere 7 cm bis 10 cm. Bevorzugt beträgt die Breite des Innenraums nicht mehr als 17 cm, besonderes bevorzugt 14 cm bis 16 cm. Die Länge der Klappe, gemessen von der sie tragenden Seite bis zu ihrem freien Klappenrand, beträgt vorzugsweise mindestens 2 cm, besonders bevorzugt 2,1 cm bis 3 cm, insbesondere 2,2 cm bis 2,8 cm.

Weitere Einzelheiten und Vorteile der Erfindung werden anhand eines Ausführungsbeispiels unter Bezugnahme auf die beigefügten Zeichnungen erläutert. Die dabei beschriebenen Merkmale können einzeln und in Kombination zum Gegenstand von Ansprüchen gemacht werden. Es zeigen:
- Figur 1: ein Ausführungsbeispiel einer erfindungsgemäßen Tasche in einer Draufsicht im geöffneten Zustand;
- Figur 2a: eine Seitenansicht zu Figur 1;
- Figur 2b: eine Seitenansicht des Ausführungsbeispiels bei unterteiltem Innenraum;
- Figur 3: einen Schnitt durch den Innenraum des Ausführungsbeispiels;
- Figur 4: eine Rückansicht zu Figur 1; und
- Figur 5: die Tasche in geschlossenem Zustand

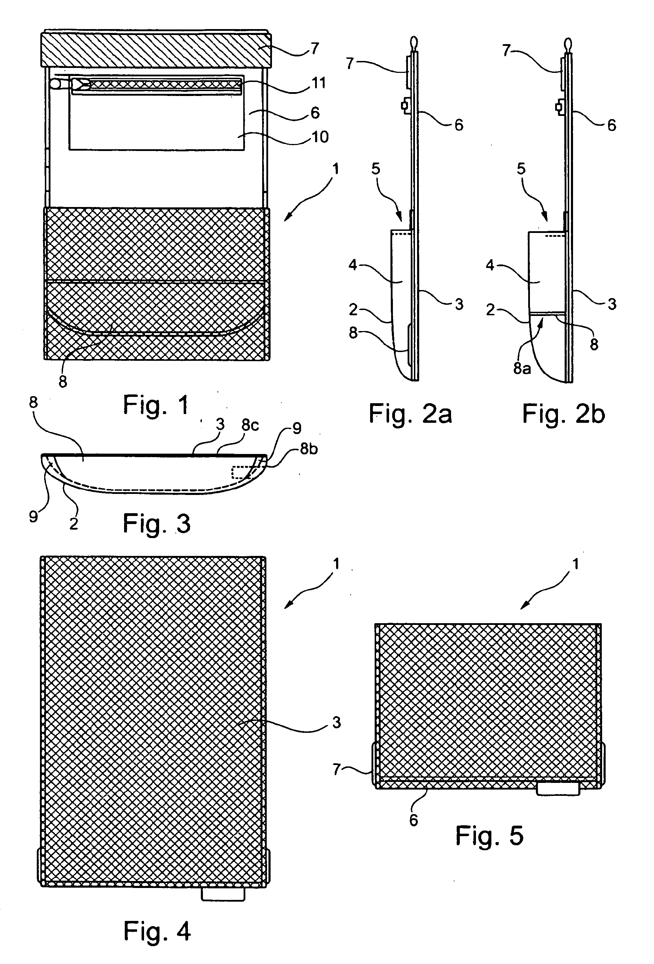

Figur 1 zeigt eine Tasche 1 für ein Blutzuckermessgerät in einer Draufsicht; Figur 2a eine dazu gehörende Seitenansicht und Figur 4 eine Rückansicht. Die Tasche 1 hat einen von einander gegenüberliegenden Seiten 2, 3 der Tasche 1 begrenzten Innenraum 4, der an einem Ende geschlossen ist und an einem gegenüberliegenden Ende eine Öffnung 5 aufweist. Ein Querschnitt durch den Innenraum 4 ist in Figur 3 gezeigt.

Die Öffnung 5 des Innenraums 4 kann mit einer Klapplasche 6 verschlossen werden, die nach dem Verschließen mit einer Schlaufe 7 fixiert werden kann. Die Klapplasche 6 ist ebenso breit wie die den Innenraum 4 begrenzende Taschenseite 3, von der sie ausgeht. Bei dem dargestellten Ausführungsbeispiel ist die Schlaufe 7 ein elastisches Textilband, das an der Klapplasche 6 angebracht ist.

Figur 5 zeigt die Tasche 1 in ihrem geschlossenem Zustand, in dem die Klapplasche 6 an einer der den Innenraum 4 begrenzenden Taschenseiten 2, 3 anliegt und, im leeren Zustand oder nur ein Blutzuckermessgerät enthaltend, die Taschenseite 2 vollständig bedeckt. Je voller die Tasche 1 ist, desto weniger weit erstreckt sich die biegsame Klapplasche 6 bei geschlossener Tasche entlang der Taschenseiten 2, 3.

Um die Klapplasche 6 bei verschlossener Öffnung 5 zu fixieren, wird das elastische Band 7 um das verschlossene Ende des Innenraums 4 herumgeführt. Möglich ist es auch, die Schlaufe 7 an einer der den Innenraum 4 begrenzenden Seitenwände 2, 3 zu befestigen. Dabei ist es nicht unbedingt erforderlich, die Schlaufe ebenso breit wie die Klapplasche 6 auszuführen, so dass dieser in die Lasche geschoben werden kann. Möglich ist es auch, die Klapplasche 6 mit einem schmaleren Fortsatz auszustatten, der in eine schmalere Schlaufe gesteckt werden kann.

Eine Besonderheit der dargestellten Tasche 1 ist eine Klappe 8, die in einem Abstand von dem geschlossenen Ende des Innenraums 4 an einer der einander gegenüberliegenden Seiten, bei dem dargestellten Ausführungsbeispiel der Seite 3, befestigt ist. In Figur 2a ist die Klappe 8 in einer ersten Stellung gezeigt, in der sie den Innenraum 4 nicht unterteilt. In Figur 2b ist die Klappe 8 in einer zweiten Stellung gezeigt, in der sie den Innenraum 4 unterteilt.

In der ersten Stellung der Klappe 8 ist eine erste Seite 8a der Klappe 8 einer der gegenüberliegenden Seiten, in Figur 2a der Seite 3, an welcher die Klappe 8 befestigt ist, zugewandt. Aus dieser ersten Stellung kann die Klappe 8 in die zweite Stellung geschwenkt werden, die in Figur 2b gezeigt ist. In der zweiten Stellung unterteilt die Klappe 8 den Innenraum 4 und ist mit ihrer ersten Klappenseite 8a dem geschlossenen Ende des Innenraums 4 zugewandt.

Bestimmungsgemäß nimmt die Klappe 8a die erste, in Figur 2a dargestellte Position, ein, wenn in der Tasche 1 ein Blutzuckermessgerät ohne einen zusätzlichen Vorrat von Verbrauchsmaterial für das Blutzuckermessgerät transportiert werden soll. Beim Einschieben des Blutzuckermessgeräts in den Innenraum 4 wird die Klappe 8 von selbst in die in Figur 2a dargestellte Position gedrückt. Soll in dem Innenraum 4 der Tasche 1 zusätzlich ein Vorrat an Verbrauchsmaterial transportiert werden, beispielsweise Testelemente, auf die eine Körperflüssigkeitsprobe zur Blutzuckermessung aufgebracht wird, Lanzetten und/oder Batterien für das Messgerät, lässt sich die Klappe 8 in die in Figur 2b dargestellte zweite Position schwenken, in der die Klappe 8 den Innenraum 4 unterteilt, so dass Verbrauchsmaterial zwischen dem geschlossenen Ende des Innenraums 4 und der Klappe 8 untergebracht und ein Blutzuckermessgerät auf einer der Öffnung 5 zugewandten Seite der Klappe 8 angeordnet werden kann. Zum Einbringen von Verbrauchsmaterial wird die Klappe 8 aus der in Figur 2a dargestellten Position um etwa 180° geschwenkt, so dass sie mit der ersten Klappenseite 8a der den Innenraum 4 begrenzenden Seite 2 zugewandt ist, die der die Klappe 8 tragenden Seite 3 gegenüberliegt.

Der Innenraum 4 der Tasche 1 hat einen D-förmigen Querschnitt, der in Figur 3 dargestellt ist. Die Klappe 8 ist näherungsweise ebenfalls D-förmig. Die Klappe 8 ist mit einem geradlinigen Klappenrand 8c befestigt, dem ein gerundeter Klappenrand 8b gegenüber liegt.

Damit sich die Klappe 8 möglichst leicht schwenken lässt, hat der Innenraum 4 des gezeigten Ausführungsbeispiels eine sich entlang der gegenüberliegenden Seiten 2, 3 erstreckende Breite, welche die sich in diese Richtung erstreckende Breite der Klappe 8 übersteigt. Zwischen den beiden Enden der Klappe 8 und der sie tragenden Seite 2 befindet sich jeweils ein kleiner Spalt 9, der bevorzugt eine Breite von 2 mm bis 5 mm hat. Der Spalt 9 erstreckt sich bei dem dargestellten Ausführungsbeispiel jedoch nur in einem Anfangsbereich der Rundung des Klappenrandes 8b. Befindet sich die Klappe 8 in der in Figur 2b gezeigten zweiten Stellung, so berührt sie beide Seiten 2, 3.

Die Klappe 8 ist steifer als die den Innenraum 4 begrenzenden Seiten 2, 3 und die Klapplasche 6. Beispielsweise können die Seiten 2, 3 und die Klapplasche 6 aus flexiblen Materialien, die beispielsweise zur Herstellung von Brieftaschen gebräuchlich sind, wie Leder, Kunstleder, Kunststoffe und/oder Textilgewebe, hergestellt sein. Für die Klappe 8 können als Außenmaterialien dieselben Materialien verwendet werden, die beispielsweise durch eine eingelegte Platte, beispielsweise aus Pappe oder Kunststoff, versteift sind. Bei dem dargestellten Ausführungsbeispiel ist die Außenseite der Seiten 2, 3 aus PCV Kunstleder, das innen mit Textilgewebe belegt ist. Zwischen diesen beiden Lagen können Zwischenlagen, beispielsweise zur Polsterung, vorhanden sein.

Bei dem dargestellten Ausführungsbeispiel berühren sich die gegenüberliegenden Seiten 2, 3, die den Innenraum 4 der Tasche 1 begrenzen, an ihren Rändern und sind dort miteinander vernäht. Die gegenüberliegenden Seiten 2, 3 und die Klapplasche 6 sind aus einem Streifen gebildet, der entlang einer das verschlossene Ende des Innenraums 4 definierenden Faltelinie gefaltet wurde, so dass ein Abschnitt seiner Seitenränder an sich selbst anliegt. auf diese Weise grenzen die gegenüberliegenden Seiten 2, 3 an dem verschlossenen Ende des Innenraums 4 fugenlos aneinander an.

In dem Deckel 6 kann ein zusätzliches Fach 10 angeordnet sein, das bei dem dargestellten Ausführungsbeispiel mit einem Reißverschluss 11 verschlossen ist.

Die vorstehend beschriebene Tasche 1 bildet zusammen mit einem Blutzuckermessgerät und Verbrauchsmaterialien für mit dem Blutzuckermessgerät durchzuführende Blutzuckermessungen ein Blutzuckermesssystem.

Die Klapplasche 6 des in den Figuren gezeigten Ausführungsbeispiels hat eine von der Öffnung 5 bis zu ihrem freien Ende gemessene Länge von 12 cm. Der Innenraum 4 von dem geschlossenen Ende bis zu der Öffnung 5 eine Länge von 10 cm. Die Breite der Öffnung 5 des Innenraums 4 beträgt 15 cm, die Breite der Klappe 8 etwa 14 cm. Die senkrecht zu Breite zu messende Länge der Klappe beträgt 2,5 cm.

### Bezugszahlen

- 1.: Tasche
- 2.: Seiten
- 3.: Seiten
- 4.: Innenraum
- 5.: Öffnung
- 6.: Klapplasche
- 7.: Schlaufe
- 8.: Klappe
- 8a.: Klappenseite
- 8b.: gerader Klappenrand
- 8c: gerundeter Klappenrand
- 9.: Spalt
- 10.: Fach
- 11.: Reißverschluss

## Patentansprüche

1. Tasche für ein Blutzuckermessgerät, mit einem von einander gegenüberliegenden Seiten (2, 3) der Tasche (1) begrenzten Innenraum (4), der an einem Ende geschlossen ist und an einem gegenüberliegenden Ende eine Öffnung (5) aufweist, **gekennzeichnet durch** eine Klappe (8), die in einem Abstand von dem geschlossenen Ende an einer der einander gegenüberliegenden Seiten (2, 3) befestigt ist und aus einer ersten Stellung, in der eine erste Seite (8a) der Klappe (8) einer der gegenüberliegenden Seiten (2, 3) zugewandt ist, in eine zweite Stellung, in welcher die Klappe (8) den Innenraum (4) der Tasche (1) unterteilt und mit der ersten Klappenseite (8a) dem geschlossenen Ende des Innenraums (4) zugewandt ist, schwenkbar ist.

2. Tasche nach Anspruch 1, **dadurch gekennzeichnet, dass** die erste Klappeseite (8a) in der ersten Stellung der Klappe (8) derjenigen der einander gegenüberliegenden Seiten (3) zugewandt ist, an der die Klappe (8) befestigt ist.

3. Tasche nach einem der vorstehenden Ansprüche, **dadurch gekennzeichnet, dass** die Klappe (8) aus einem steiferen Material als die den Innenraum (4) begrenzenden Seiten (2, 3) ist.

4. Tasche nach einem der vorstehenden Ansprüche, **dadurch gekennzeichnet, dass** die Klappe (8) einen gerundeten Klappenrand (8b) hat.

5. Tasche nach einem der vorstehenden Ansprüche, **dadurch gekennzeichnet, dass** sich die gegenüberliegenden Seiten (2, 3), die den Innenraum (4) der Tasche (1) begrenzen, an ihren Rändern berührten.

6. Tasche nach einem der vorstehenden Ansprüche, **dadurch gekennzeichnet, dass** der Innenraum (4) der Tasche (1) und sein Öffnung (5) eine sich entlang der einander gegenüberliegenden Seiten (2, 3) erstreckende Breite hat, welche die sich in dieser Richtung erstreckende Breite der Klappe (8) übersteigt.

7. Tasche nach einem der vorstehenden Ansprüche, **dadurch gekennzeichnet, dass** die einander gegenüberliegenden Seiten (2, 3) an dem verschlossenen Ende des Innenraums (4) fugenlos aneinander angrenzen.

8. Tasche nach einem der vorstehenden Ansprüche, **gekennzeichnet durch** eine Klapplasche (6), die zum Verschließen des Innenraums (4) über dessen Öffnung (5) geklappt wird.

9. Tasche nach Anspruch 8, **dadurch gekennzeichnet, dass** die Klapplasche (6) von der Taschenseite (3) ausgeht, an der die Klappe (8) befestigt ist.

10. Tasche nach Anspruch 8 oder 9, **dadurch gekennzeichnet, dass** die Klapplasche (6) in verschiedenen Schließstellungen über die Öffnung (5) klappbar ist, die sich darin unterscheiden, wie weit sich die Klapplasche (6) entlang der einander gegenüberliegenden Seiten (2, 3) erstreckt.

11. Blutzuckermesssystem mit
einem Blutzuckermessgerät,
Verbrauchsmaterial für mit dem Blutzuckermessgerät durchzuführende Blutzuckermessungen und
einer Tasche (1) nach einem der vorstehenden Ansprüche zur Aufnahme der Verbrauchsmittel in dem Innenraum (4) der Tasche (1) zwischen dessen verschlossenem Ende und der in der zweiten Stellung angeordneten Klappe (8) sowie der Aufnahme des Blutzuckermessgeräts auf einer der Öffnung (5) zugewandten Seite der Klappe (8).

## Claims

1. Carry case for a blood-sugar measuring device, the carry case having opposing sides (2, 3) delimiting an interior space (4) that is closed at one end and provided with an opening (5) at the other end, **characterized by** a flap (8) that is fastened at a distance from the closed end at one of the opposing sides (2, 3) and that can be swung from a first position in which a first side (8a) of the flap (8) faces one of the opposing sides (2, 3) to a second position, in which the flap (8) divides the interior space (4) of the carry case (1) and faces with the first flap side (8a) the closed end of the interior space (4).

2. Carry case according to claim 1, **characterized in that** the first flap side (8a) in the first position of the flap (4) is facing the one of the opposing sides (3) at which the flap (8) is fastened.

3. Carry case according to any of above claims, **characterized in that** the flap (8) is made out of a stiffer material than the sides (2, 3) delimiting the inside (4).

4. Carry case according to any of above claims, **characterized in that** the flap (8) has a rounded flap edge (8b).

5. Carry case according to any of above claims, **characterized in that** the opposing sides (2, 3) that delimit the interior space (4) of the carry case (1) touch each other with their edges.

6. Carry case according to any of above claims, **characterized in that** the interior space (4) of the carry case (1) and its opening (5) have a width extending along the opposing sides (2, 3) that exceeds the width of the flap (8) measured in this direction.

7. Carry case according to any of above claims, **characterized in that** the opposing sides (2, 3) abut each other seamlessly at the closed end of the inside (4).

8. Carry case according to any of above claims, **characterized by** a strap (6) that is folded over the opening of the interior space (4) in order to close it.

9. Carry case according to claim 8, **characterized in that** the strap (6) starts at the side (3) of the carry case at which the flap (8) is fastened.

10. Carry case according to claim 8 or 9, **characterized in that** the strap (6) can be folded over the opening (5) in several closing positions, that differ with respect to the extent the strap (6) extends along the opposing sides (2, 3).

11. Blood-sugar measuring system with
a blood-sugar measuring device,
consumable material for the blood measurements to be effectuated with the blood-sugar measuring device and
a carry case (1) according to any of above claims for the storing of consumable material in the interior space (4) of the carry case (1) between its closed end and the flap (8) in its second position, as well as for the storing of the blood-sugar measuring device a side of the flap (8) facing the opening (5).

## Revendications

1. Poche pour un lecteur de glycémie, dotée d'un espace intérieur (4) délimité par des côtés (2, 3) opposés de la poche (1), qui est fermé sur une extrémité et présente une ouverture (5) sur une extrémité opposée, **caractérisée par** un clapet (8), qui est fixé à une distance de l'extrémité fermée sur l'un des côtés (2, 3) opposées et peut être basculé à partir d'une première position, dans laquelle un premier côté (8a) du clapet (8) est tourné vers l'un des côtés (2, 3) opposés, dans une seconde position dans laquelle le clapet (8) subdivise l'espace intérieur (4) de la poche (1) et est tourné avec le premier côté du clapet (8a) vers l'extrémité fermée de l'espace intérieur (4).

2. Poche selon la revendication 1, **caractérisée en ce que** le premier côté de clapet (8a) est tourné, dans la première position du clapet (8), vers l'un des côtés (3) opposés sur lequel le clapet (8) est fixé.

3. Poche selon l'une quelconque des revendications précédentes, **caractérisée en ce que** le clapet (8) est à base d'un matériau plus rigide que les côtés (2, 3) délimitant l'espace intérieur (4).

4. Poche selon l'une quelconque des revendications précédentes, **caractérisée en ce que** le clapet (8) a un bord de clapet (8b) arrondi.

5. Poche selon l'une quelconque des revendications précédentes, **caractérisée en ce que** les côtés (2, 3) opposés, qui délimitent l'espace intérieur (4) de la poche (1) se touchent sur leurs bords.

6. Poche selon l'une quelconque des revendications précédentes, **caractérisée en ce que** l'espace intérieur (4) de la poche (1) et son ouverture (5) ont une largeur s'étendant le long des côtés (2, 3) opposés qui dépasse la largeur du clapet (8) s'étendant dans cette direction.

7. Poche selon l'une quelconque des revendications précédentes, **caractérisée en ce que** les côtés (2, 3) opposés se touchent sans joint sur l'extrémité fermée de l'espace intérieur (4).

8. Poche selon l'une quelconque des revendications précédentes, **caractérisée par** une patte rabattable (6) qui, pour fermer l'espace intérieur (4), est rabattue sur l'ouverture (5) de cet espace.

9. Poche selon la revendication 8, **caractérisée en ce que** la patte rabattable (6) part du côté de poche (3) sur lequel le clapet (8) est fixé.

10. Poche selon la revendication 8 ou 9, **caractérisée en ce que** la patte rabattable (6) peut se rabattre sur l'ouverture (5) dans différentes positions de fermeture qui se différencient par la largeur sur laquelle la patte rabattable (6) s'étend le long des côtés (2, 3) opposés.

11. Système lecteur de glycémie comprenant
un lecteur de glycémie,
du matériau consommable pour les mesures de glycémie effectuées avec le lecteur de glycémie et
une poche (1) selon l'une quelconque des revendications précédentes pour le logement des produits consommables dans l'espace intérieur (4) de la poche (1) entre l'extrémité fermée de l'espace intérieur et le clapet (8) disposé dans la seconde position et pour le logement du lecteur de glycémie sur un côté du clapet (8) tourné vers l'ouverture (5).
